# EUROPEAN PATENT SPECIFICATION

(11) **EP 0 628 039 B1**
(45) Date of publication and mention of the grant of the patent: **02.05.1997**
(21) Application number: 93905215.5
(22) Date of filing: 05.02.1993
(51) Int. Cl.: C07D 405/04, A61K 31/55

(54) **2,3,4,5-TETRAHYDRO-1H-3-BENZAZEPINES AND PHARMACEUTICALLY ACCEPTABLE ACID ADDITION SALTS THEREOF**
2,3,4,5-TETRAHYDRO-1H-3-BENZAZEPINE UND IHRE PHARMAZEUTISCH VERTRÄGLICHEN SÄUREADDITIONSSALZE
2,3,4,5-TETRAHYDRO-1H-3-BENZAZEPINES ET LEURS SELS D'ADDITION D'ACIDES ACCEPTABLES SUR LE PLAN PHARMACEUTIQUE

(30) Priority: 24.02.1992 DK 233/92
(43) Date of publication of application: 14.12.1994
(73) Proprietor: NOVO NORDISK A/S, 2880 Bagsvaerd (DK)
(72) Inventor: FOGED, Christian, Dk-3460 Birker d (DK); HOHLWEG, Rolf, Dk-3490 Kvistgaard (DK); NIELSEN, Erik, Bardrum, Dk-3500 V rl se (DK)
(74) Representative: Madsen, Inger Margrethe Schelde
(86) International application number: DK9300041
(87) International publication number: WO9317012

(56) References cited:
- EP-A- 0 200 455
- EP-A- 0 347 672
- EP-A- 0 383 247

## Description

This invention relates to novel 2,3,4,5-tetrahydro-1H-3-benzazepines and pharmaceutically acceptable acid addition salts thereof, to methods for their preparation, to pharmaceutical compositions containing them, and to their use in the treatment of certain disorders in the central nervous system, e.g., psychosis, pain, depression, sleep disturbances, dyskinesias, Parkinson's disease, stroke.

In the last decade intensive pharmacological research concerning benzazepines has taken place. The pharmacological properties of benzazepines depend to a large extent on the substituents. Various substituted benzazepines exhibiting neuroleptic, anti-aggressive, anti-Parkinson and vascular effects are known.

In European Patent No. 0 200 455 (Novo Industri A/S) 2,3,4,5-tetrahydro-1H-3-benzazepines having a heterocyclic or an ortho-fused heterocyclic ringsystem in the 5-position are described. These compounds are claimed to have antipsychotic and antidepressive effects.

In European Patent No. 0347672 2,3,4,5-tetrahydro-1H-3-benzazepines having phenyl or a fused phenyl group in the 5-position and a nitro group in position 8 are described. The benzazepines are stated to be useful as neuroleptics in the treatment of various mental disorders, e.g. manic-depressive disorders.

It has now been found that a group of 5'- or 6'-substituted or 5',6'-disubstituted (2,3-dihydrobenzofuran-7-yl) -2,3,4,5-tetrahydro-1H-3-benzazepine compounds exhibit strong antidopaminergic effect which makes them useful in psychopharmaceutical applications.

Surprisingly, the compounds of the invention exhibit unexpectedly high oral antidopaminergic activity compared to known compounds.

According to the present invention there are provided 2,3,4,5-tetrahydro-1H-3-benzazepines of the general formula I 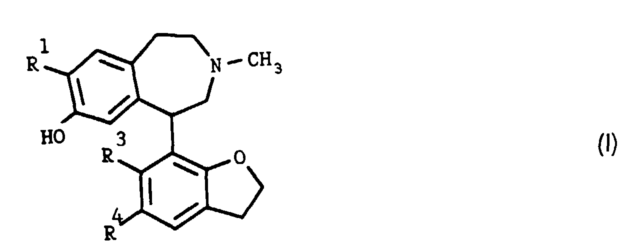 wherein R¹ is Cl or Br;
R³ and R⁴ independently are hydrogen, halogen, CF₃, CN, NO₂ or NH₂ and pharmaceutically acceptable acid addition salts thereof, provided that R³ and R⁴ cannot be hydrogen at the same time.

Specific compounds of formula (I) are:
(+) 8-chloro-5-(5-bromo-2,3-dihydrobenzofuran-7-yl)-7-hydroxy-3-methyl-2,3,4,5-tetrahydro-1H-3-benzazepine,
(+) 8-chloro-5-(2,3-dihydro-5-iodo-benzofuran-7-yl)-7-hydroxy-3-methyl-2,3,4,5-tetrahydro-1H-3-benzazepine,
(+) 8-chloro-5-(5,6-dichloro-2,3-dihydrobenzofuran-7-yl)-7-hydroxy-3-methyl-2,3,4,5-tetrahydro-1H-3-benzazepine,
(+) 8-chloro-5-(5-chloro-2,3-dihydrobenzofuran-7-yl)-7-hydroxy-3-methyl-2,3,4,5-tetrahydro-1H-3-benzazepine,
8-chloro-5-(5-nitro-2,3-dihydrobenzofuran-7-yl)-7-hydroxy-3-methyl-2,3,4,5-tetrahydro-1H-3-benzazepine,
8-chloro-5-(5-amino-2,3-dihydrobenzofuran-7-yl)-7-hydroxy-3-methyl-2,3,4,5-tetrahydro-1H-3-benzazepine

The compounds of formula I may be presented as a mixture of enantiomers, which may be resolved into the individual pure enantiomers. This resolution may conveniently be performed by fractional crystallization from various solvents, of the salts of compounds of the formula I with optical active acids or by other methods known from the literature, e.g. chiral column chromatography. Therefore, this invention includes all isomers, whether resolved or mixtures thereof.

Particularly valuable embodiments of this invention are non-toxic, pharmaceutically acceptable acid addition salts of benzazepines of formula I. Such salts include those derived from inorganic and organic acids such as hydrochloric, hydrobromic, sulphuric, phosphoric, methanesulfonic, acetic, lactic, maleic, phthalic and tartaric acids.

These salts may be prepared by methods known to professionals skilled in the art.

The invention also relates to compounds of formula I, wherein R³ or R⁴ is a radioactive isotope of iodine or bromine, such as the clinically used isotopes, ¹²²I, ¹²³I, ^{l25}I, ¹³¹I, ⁷⁷Br, ⁸²Br and ⁷⁶Br. These compounds have been found useful as imaging agents in Single Photon Emission Computed Tomography (SPECT) or in Positron Emission Tomography (PET).

The invention further provides pharmaceutical compositions comprising the compounds of the invention. The dosage formulation will preferably contain the active compounds in the range of 0.1 mg to about 1000 mg for oral dosing. Typical dosage for antipsychotic effect would vary between about 0.5 to 10 mg/kg per day divided in 2 or 3 doses, administered orally.

The compounds of the present invention can be prepared by various methods. These methods comprise:
a) halogenation of a compound of formula II 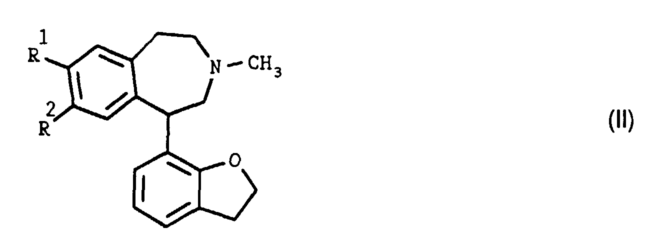 wherein R¹ is defined as above and R² is O-C₁₋₄-alkyl or O-CO-C₁₋₄-alkyl to form a compound of the general formula III 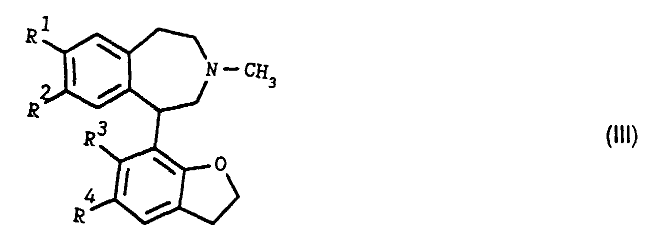 wherein R¹ and R² are defined as above, R³ is halogen or H, R⁴ is halogen, and deacylation or dealkylation of a compound of formula III to form a compound of formula I, wherein R¹, R³ and R⁴ are defined as above,
b) nitration of a compound of formula II 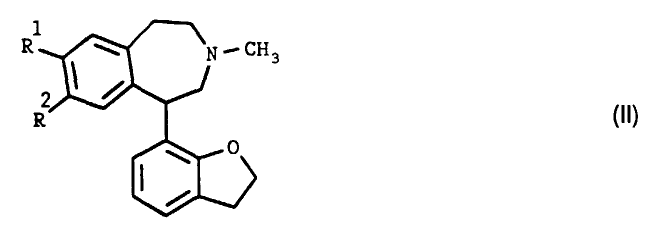 as defined in a) to form a compound of the general formula III, 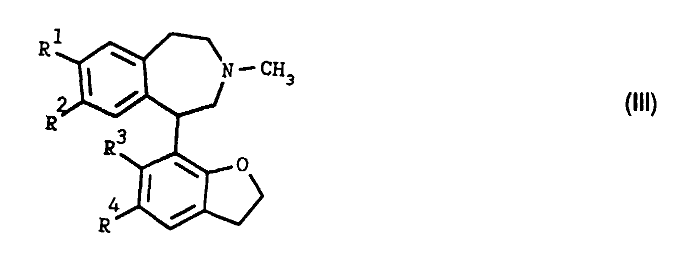 wherein R¹ and R² are defined as in a), R³ is H and R⁴ is -NO₂, and deacylation or dealkylation of a compound of formula III to form a compound of formula I, wherein R¹, R³ and R⁴ are as defined above or
c) reduction or catalytic hydrogenation of a compound of formula IV, 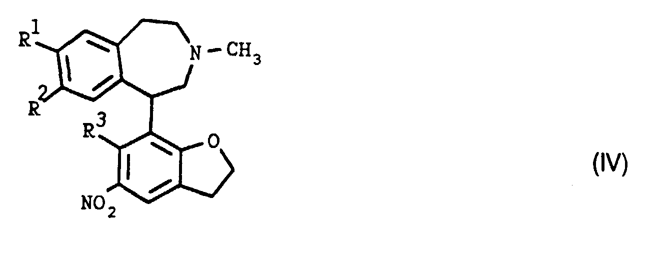 wherein R¹ is defined as above, R² is O-C₁₋₄-alkyl or O-CO-C₁₋₄-alkyl and R³ is H, to a compound of formula III, 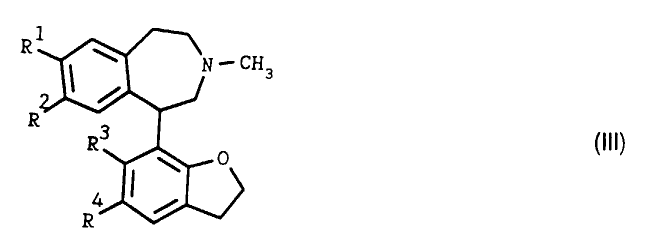 wherein R¹, R² and R³ are defined as above and R⁴ is -NH₂, and deacylation or dealkylation of the compound of formula III to form a compound of formula I, wherein R¹, R³ and R⁴ are defined as above.

The starting materials employed in the synthesis of the compounds of formula I are known e.g. from European Patent No. EP 0.200.455.

The compounds of the invention are useful because of their pharmacological activity. In particular, the compounds of the invention are active in assays predictive for antipsychotic effect. Thus the compounds of formula I were tested for their binding to dopamine D₁ receptor in homogenates from rat striatum using the method described (Life Science vol. 37, p. 1971 (1985) P. Andersen et al.) and the result appears from Table I. IC₅₀ is the affinity of tested compounds for the dopamine D₁ receptor.

**TABLE I**

| Test Compound | IC₅₀ (nM) Dopamine D₁ receptor |
|---|---|
| Example No. 1 | 0.7 |
| Example No. 3 | 0.9 |

The previously mentioned high oral antidopaminergic activity of the compounds of the present invention in comparison to known compounds without the claimed 5'-substituents or 6'-substituents (EP 200.455) can be shown by calculating the ratio between oral ability to inhibit stereotyped behaviour in mouse following methylphenidate (i.e., Acta Pharmacol. Toxicol. 31, 1972, 488) and inhibition of ³H-SCH 23390 binding in vitro (measure of D₁-receptor antagonism). This yielded the following ratios:

| Test Compound | Index of oral mg/kg / IC₅₀ SCH 23390 binding |
|---|---|
| Example no. 1 | 4 |
| Example no. 3 | 1.6 |
| Example no. 5 in EP 200.455 | 23 |

The compound of the invention, together with a conventional adjuvant, carrier, or diluent, and if desired in the form of a pharmaceutically acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form or sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective central nervous system ailment alleviating amount of the active ingredient commensurate with the intended daily dosage range to be employed. Tablets containing 0.1-1000 mg of active ingredient or, more specified 0.5-10 mg, per tablet, are accordingly suitable representative unit dosage forms.

The compounds of this invention can thus be used for the formulation of pharmaceutical preparations, e.g., for oral and parenteral administration to mammals including humans, in accordance with conventional methods of galenic pharmacy.

Conventional excipients are such pharmaceutically acceptable organic or inorganic carrier substances suitable for parenteral or oral application which do not deleteriously react with the active compound.

Examples of such carriers are water, salt solutions, alcohols, polyethylene glycols, polyhydroxyethoxylated castor oil, syrup, peanut oil, olive oil, gelatin, lactose, terra alba, sucrose, agar, pectin, acacia, amylose, magnesium stearate, talc, silicic acid, stearic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, such as lubricants, preservatives, stabilizers, wetting agents, emulsifiers, salt for influencing osmotic pressure, buffers and/or colouring substances and the like, which do not deleteriously react with the active compound.

For parenteral application, particularly suitable are injectable solutions or suspensions, preferably aqueous solutions with the active compound dissolved in polyhydroxylated castor oil.

Ampoules are convenient unit dosage forms.

For oral administration, particularly suitable are tablets, dragees, or capsules having talc and/or a carbohydrate carrier or binder or the like, the carrier preferably being lactose and/or corn starch and/or potato starch. A syrup, elixir or like can be used when a sweetened vehicle can be employed. Generally, as to broader ranges, the compounds of the invention are dispensed in unit dosage form comprising 0.05-100 mg in a pharmaceutically acceptable carrier per unit dosage.

A typical tablet, which may be prepared by conventional tabletting techniques, contains:

| | |
|---|---|
| Active compound | 1.0 mg |
| Lactosum | 67.8 mg Ph.Eur. |
| Avicel® | 31.4 mg |
| Amberlite® IRP 88 | 1.0 mg |
| Magnesii stearas | 0.25 mg Ph.Eur. |

The following examples illustrate the preparation of the novel compounds of this invention:

### EXAMPLE 1

### (+) 8-Chloro-5-(5-bromo-2,3-dihydrobenzofuran-7-yl)-7-hydroxy-3-methyl-2,3,4,5-tetrahydro-1H-3-benzazepine

a) (+) 8-Chloro-5-(2,3-dihydrobenzofuran-7-yl)-7-methoxy-3-methyl-2,3,4,5-tetrahydro-1H-3-benzazepine (1.0 g, 2.9 mmol) was dissolved in acetic acid (10 ml). To the stirred solution was added bromine (0.20 ml, 4.0 mmol) in acetic acid (5 ml) over a period of 2 h. The mixture was stirred overnight at room temperature. A precipitate was formed. The white solid was filtered and washed with diethylether.
   Yield 1.1 g (75%) of (+) 8-chloro-5-(5-bromo-2,3-dihydrobenzufuran-7-yl)-7-methoxy-3-methyl-2,3,4,5-tetrahydro-1H-3-benzazepine, HBr as a white crystalline powder.
   NMR 200 MHz¹H-chemicae shifts in ppm of the free base. CDCl₃ as solvent, TMS as internal standard.
   [δ, ppm]: 2.42 (s,3H), 2.40-2.55 (m,1H), 2.95 (m,5H), 3.28 (t,2H), 3.70 (s,3H), 4.43 (d,1H), 4.58 (t,2H), 6.38 (s,1H), 6.98 (d,1H), 7.18 (s,1H), 7.26 (d,1H).
b) (+) 8-Chloro-5-(5-bromo-2,3-dihydrobenzofuran-7-yl)-7-methoxy-3-methyl-2,3,4,5-tetrahydro-1H-3-benzazepine (3.9 g, 9.2 mmol) was dissolved in dichloromethane (50 ml). The solution was cooled on an icebath. To the stirred solution was added 10 vol % BBr₃ in dichloromethane (14 ml, 14.8 mmol). The reaction mixture was stirred for 2 h, and then warmed to room temperature. The mixture was added methanol and concentrated at reduced pressure. Methanol was added (50 ml) and the mixture was refluxed for 2 h, and then stirred at room temperature overnight. The mixture was concentrated to a thick oil. Methanol (15 ml) was added and under stirring NaOH (0.5 M) was added until precipitation started. The mixture was stirred for one hour, then cooled on an icebath. The brown solid was filtered and washed with water.
   Yield 3.2 g (85%) of the title compound as a crystalline slightly brown powder.
   NMR 200 MHz ¹H-chemicae shifts in ppm. of the free base. CDCl₃ as solvent, TMS as internal standard.
   [δ, ppm]: 2.25 (t,1H), 2.35 (s,3H), 2.90 (m,5H), 3.22 (t,2H), 4.35 (d,1H), 4.51 (t,2H), 6.30 (s,1H), 6.96 (d,1H), 7.10 (s,1H), 7.22 (d,1H).

### EXAMPLE 2

### (+) 8-Chloro-5-(2,3-dihydro-5-iodo-benzofuran-7-yl)-7-hydroxy-3-methyl-2,3,4,5-tetrahydro-1H-3-benzazepine

a) Iodine (4.2 g, 16.3 mmol) and 32% peracetic acid (10.3 ml, 49 mmol) was stirred in acetic acid (50 ml) for 15 min. at room temperature. (+) 8-Chloro-5-(2,3-dihydrobenzofuran-7-yl)-7-methoxy-3-methyl-2,3,4,5-tetrahydro-1H-3-benzazepine (5.6 g, 16.3 mmol) in acetic acid (50 ml) was added to the mixture over a period of 1 h. The mixture was stirred for additional 3 h. The mixture was treated with sodium thiosulfate and evaporated under reduced pressure to a brown solid. Dichloromethane was added and the organic phase was washed with water, NaOH (aq), water and then dried over anhydrous magnesium sulfate. The extract was filtered and concentrated to a brown solid. The product was purified by column chromatography (silica gel: CH₂Cl₂-MeOH).
   Yield: 4.8 g (63%) of (+) 8-chloro-5-(2,3-dihydro-5-iodo-benzofuran-7-yl)-7-methoxy-3-methyl-2,3,4,5-tetrahydro-1H-3-benzazepine as a crystalline slightly brown powder.
   NMR 200 MHz ¹H-chemicae shifts in ppm of the free base. CDCl₃ as solvent, TMS as internal standard.
   [δ, ppm]: 2.32 (m,1H), 2.36 (s,3H), 2.92 (m,5H), 3.22 (t,2H), 3.70 (s,3H), 4.37 (dd,1H), 4.52 (t,2H), 6.35 (s,1H), 7.13 (broad s,2H), 7.42 (d,1H).
b. (+) 8-chloro-5-(2,3-dihydro-5-iodo-benzofuran-7-yl)-7-methoxy-3-methyl-2,3,4,5-tetrahydro-1H-3-benzazepine (300 mg, 0.65 mmol) was dissolved in dichloromethane (15 ml). To the stirred solution was added 10% BBr₃ in dichloromethane (2.0 ml, 2.1 mmol) over a period of 1 h. The mixture was stirred at room temperature for additionally 15 min. The mixture was diluted with methanol (25 ml) and concentrated at reduced pressure. Methanol (20 ml) was added, and under stirring NaOH (0.5 M) was added until pH 7. The mixture was stirred for 1 h and water was added until precipitation started. The solid was filtered and washed with water.
   Yield: 207 mg (70%) of the title compound as a crystalline slightly brown powder.
   NMR 200 MHz ¹H-chemicae shifts in ppm of the free base. CDCl₃ as solvent, TMS as internal standard.
   (δ, ppm): 2.28 (t,1H), 2.38 (s,3H), 2.90 (m,5H), 3.22 (t,2H), 4.37 (d,1H), 4.52 (t,2H), 6.30 (s,1H), 7.10 (s,1H), 7.18 (d,1H), 7.40 (d,1H).

### EXAMPLE 3

### (+)8-Chloro-5-(5,6-dichloro-2,3-dihydrobenzofuran-7-yl)-7-hydroxy-3-methyl-2,3,4,5-tetrahydro-1H-3-benzazepine

1.65 g (0.005 mole) (+)8-chloro-5-(2,3-dihydrobenzofuran-7-yl)-7-hydroxy-3-methyl-2,3,4,5-tetrahydro-1H-3-benzazepine was dissolved in 2.5 ml acetic anhydride and left at room temperature for 1 h. The clear solution was concentrated in vacuo and repeatedly stripped with glacial acetic acid. Then the residue was redissolved in 10 ml acetic acid and 7.2 ml (0.0055 mole) of a 0.77 molar solution of chlorine in glacial acetic acid was slowly added to the stirred solution at room temperature. After 1 h another 7.1 ml of the chlorine solution was added. The reaction mixture was again stirred for 1 h. Then 20 ml ethanol and 1 ml concentrated HCl were added, and the mixture was refluxed for 1 h. After cooling, the solution was diluted with 20 ml ethanol and adjusted to pH 8 by careful addition of 10% Na₂CO₃ solution.

The crude product precipitated out spontaneously and was collected by filtration. Column chromatography (stationary phase: C18 silica; eluent: ammoniumsulfate/acetonitrile 70:30; pH 3.3) yielded the pure title compound. M.p.: 224-225°C.

¹H-NMR in CDCl₃ [δ, ppm]: 2.23 (t,1H); 2.40 (s,3H); 2.73 (dd,1H); 2.80-3.37 (m,6H); 4.57 (t,2H); 4.93 (d,1H); 6.17 (broad s,1H); 7.07 (s,1H); 7.25 (s,2H).

### EXAMPLE 4

### (+)8-Chloro-5-(5-chloro-2,3-dihydrobenzofuran-7-yl)-7-hydroxy-3-methyl-2,3,4,5-tetrahydro-1H-3-benzazepine

The synthesis and workup followed essentially the method described in example 3, with the only difference that 1.1 equivalent of chlorine was used for the chlorination.

The crude product was chromatographically purified as described above and yielded the free base as a crystalline powder. M.p.: 182-186°C.

¹H-NMR in CDCl₃ [δ, ppm] 2.40 (s,3H); 2.30-3.20 (m,8H); 3.90 (dd,1H); 4.15 (t,2H); 5.96 (s,1H); 6.40 (d,1H); 6.65 (s,1H); 6.70 (d,1H).

### EXAMPLE 5

### 8-Chloro-5-(5-nitro-2,3-dihydrobenzofuran-7-yl)-7-hydroxy-3-methyl-2,3,4,5-tetrahydro-1H-3-benzazepine

a) 1.50 g (0.00386 mole) 8-chloro-5-(2,3-dihydrobenzofuran-7-yl)-7-methoxy-3-methyl-2,3,4,5-tetrahydro-1H-3-benzazepine was dissolved in 20 ml acetic acid. 2.80 g (0.0116 mole) copper nitrate trihydrate was added and the mixture was stirred at room temperature for 18 h. The solvent was evaporated in vacuo, the residue treated with aqueous ammonia and extracted with dichloromethane. The solution was washed with water and brine, concentrated in vacuo and the residue redissolved in ether. Unsoluble impurities were removed by filtration and 8-chloro-5-(5-nitro-2,3-dihydrobenzofuran-7-yl)-7-methoxy-3-methyl-2,3,4,5-tetrahydro-1H-3-benzazepine was obtained as the hydrochloride by adding an excess of a HCI solution in ether and collecting the precipitate by filtration. White crystals. M.p.: 251-254°C.
   ¹H-NMR of the free base in CDCl₃ [δ, ppm]: 2.37 (s,3H); 2.40-3.25 (m,6H); 3.34 (t,2H); 3.70 (s,3H); 4.45 (d,1H); 4.73 (t,2H); 6.36 (s,1H); 7.20 (s,1H); 7.86 (d,1H); 8.03 (d,1H).
b) 0.220 g (0.00052 mole) 8-chloro-5-(5-nitro-2,3-dihydrobenzofuran-7-yl)-7-methoxy-3-methyl-2,3,4,5-tetrahydro-1H-3-benzazepine hydrochloride was dissolved in 10 ml dry dichloromethane and cooled in an ice bath. To the stirred solution 5 ml of a 1 molar solution of boron trichloride in hexane was added. The reaction mixture was allowed to warm up to room temperature and was stirred for 4.5 h. Then the solution was cooled in an ice bath and hydrolized by carefully adding 15 ml methanol. The formed crystalline hydrochloride of the title compound was collected by filtration and dried.
Slightly pink crystalline powder. M.p.: 265-270°C under decomposition.
   ¹H-NMR in d₆-DMSO [δ, ppm]: 2.80 (d,3H); 2.85-3.85 (m,8H); 4.75 (t,2H); 4.90 (d,1H); 6.22 (s,1H); 7.27 (s,1H); 8.02 (d,1H); 8.25 (d,1H); 9.95 (s,1H); 11.30 (broad s,1H).

### EXAMPLE 6

### 8-Chloro-5-(5-amino-2,3-dihydrobenzofuran-7-yl)-7-hydroxy-3-methyl-2,3,4,5-tetrahydro-1 H-3-benzazepine

a) A stirred mixture of 2.50 g (0.011 mole) sodium sulfide and 0.60 g (0.011 mole) ammonium chloride in 10 ml n-propanol was warmed to reflux temperature and a solution of 0.44 g (0.0011 mole) 8-chloro-5-(5-nitro-2,3-dihydrobenzofuran-7-yl)-7-methoxy-3-methyl-2,3,4,5-tetrahydro-1H-3-benzazepine was added dropwise and reflux was continued for 16 h. The solvent was removed in vacuo and the residue was redissolved in dichloromethane. The solution was washed with 0.1 N NaOH, water and brine. Evaporation yielded 8-chloro-5-(5-amino-2,3-dihydrobenzofuran-7-yl)-7-methoxy-3-methyl-2,3,4,5-tetrahydro-1H-3-benzazepine as a foam.
   ¹H-NMR in CDCl₃ [δ, ppm]: 2.37 (s,3H + m,1H); 2.75-3.30 (m,7H); 3.35 (broad s,2H); 3.68 (s,3H); 4.33 (t,1H); 4.46 (t,2H); 6.21 (d,1H); 6.43 (s,1H); 6.53 (d,1H); 7.12 (s,1H).
b) 2.8 ml (0.0056 mole) of a 2M solution of boron trichloride in dichloromethane was added to an icecold stirred solution of 200 mg (0.00056 mole) of the product of step a. in 10 ml dichloromethane. The reaction mixture was stirred for 8 h at room temperature and was then concentrated in vacuo. The residue was redissolved in ether, washed with saturated NaHCO₃-solution, water and brine and dried over Na₂SO₄. Precipitation with a solution of HCl in ether yielded the title compound as the hydrochloride. M.p.: 241-244°C.
   ¹H-NMR in d₆-DMSO [δ, ppm]: 2.80 (s,3H); 3.00 (m,2H); 3.31 (t,2H); 3.35-3.67 (m,4H); 4.60 (t,2H); 4.77 (d,1H); 6.23 (s,1H); 6.98 (s,1H); 7.26 (s,1H); 7.33 (s,1H); 10.00 (s,1H); 10.25 (broad s,2H); 10.00 (s,1H).

## Claims

1. 2,3,4,5-tetrahydro-1H-3-benzazepines having the general formula I 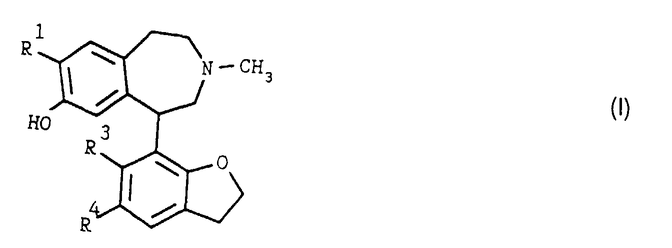 wherein R¹ is Cl or Br;
R³ and R⁴ independently are hydrogen, halogen, CF₃, CN, NO₂ or NH₂ and pharmaceutically acceptable acid addition salts thereof, provided that R³ and R⁴ cannot be hydrogen at the same time.

2. Compounds according to claim 1 wherein R¹ is Cl.

3. A compound according to claim 1 which is (+)-8-chloro-5-(5-bromo-2,3-dihydrobenzofuran-7-yl)-7-hydroxy-3-methyl-2,3,4,5-tetrahydro-1H-3-benzazepine,
(+)-8-chloro-5(2,3-dihydro-5-iodo-benzofuran-7-yl)-7-hydroxy-3-methyl-2,3,4,5-tetrahydro-1H-3-benzazepine,
(+) 8-chloro-5(5,6-dichloro-2,3-dihydrobenzofuran-7-yl)-7-hydroxy-3-methyl-2,3,4,5-tetrahydro-1H-3-benzazepine,
(+) 8-chloro-5(5-chloro-2,3-dihydrobenzofuran-7-yl)-7-hydroxy-3-methyl-2,3,4,5-tetrahydro-1H-3-benzazepine,
8-chloro-5(5-nitro-2,3-dihydrobenzofuran-7-yl)-7-hydroxy-3-methyl-2,3,4,5-tetrahydro-1H-3-benzazepine or
8-chloro-5(5-amino-2,3-dihydrobenzofuran-7-yl)-7-hydroxy-3-methyl-2,3,4,5-tetrahydro-1H-3-benzazepine.

4. A pharmaceutical composition comprising a compound according to claims 1-3 or a pharmaceutically acceptable acid addition salt thereof and a pharmaceutically acceptable carrier or diluent.

5. A pharmaceutical composition suitable for use in the treatment of a central nervous system ailment comprising a compound according to claims 1-3 together with a pharmaceutically acceptable carrier or diluent.

6. A pharmaceutical composition according to claim 4 or 5 wherein it is in the form of an oral dosage unit containing 0.1-100 mg of the active compound.

7. Use of a compound according to claims 1-3 for producing a pharmaceutical composition for the treatment of an indication related to a central nervous system ailment.

8. A method for the preparation of a compound according to claim 1, characterized in
a) halogenation of a compound of formula II 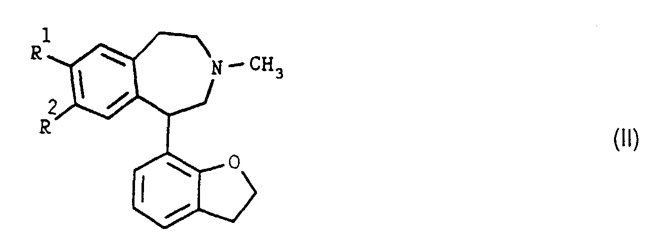 wherein R¹ is defined as above and R² is O-C₁₋₄-alkyl or O-CO-C₁₋₄-alkyl to form a compound of the general formula III 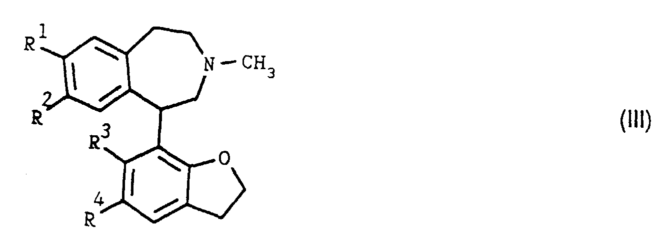 wherein R¹ and R² are defined as above, R³ is halogen or H, R⁴ is halogen, and deacylation or dealkylation of a compound of formula III to form a compound of formula I, wherein R¹, R³ and R⁴ are defined as above,
b) nitration of a compound of formula II 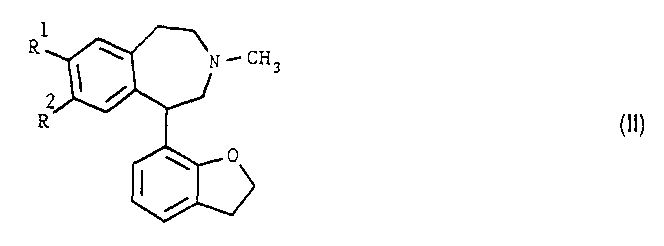 as defined in a) to form a compound of the general formula III, 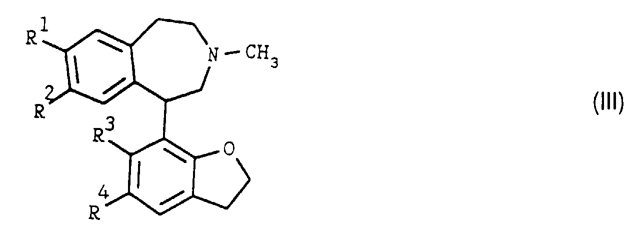 wherein R¹ and R² are defined as in a), R³ is H and R⁴ is -NO₂, and deacylation or dealkylation of a compound of formula III to form a compound of formula I, wherein R¹, R³ and R⁴ are as defined above or
c) reduction or catalytic hydrogenation of a compound of formula IV, 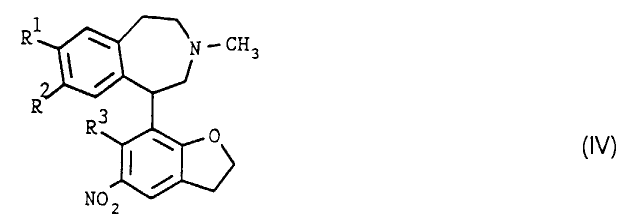 wherein R¹ is defined as above, R² is O-C₁₋₄-alkyl or O-CO-C₁₋₄-alkyl and R³ is H, to a compound of formula III, 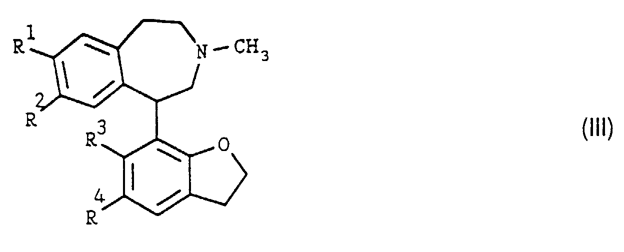 wherein R¹, R² and R³ are defined as above and R⁴ is -NH₂, and deacylation or dealkylation of the compound of formula III to form a compound of formula I, wherein R¹, R³ and R⁴ are defined as above.

## Patentansprüche

1. 2,3,4,5-Tetrahydro-1H-3-benzazepine der allgemeinen Formel I 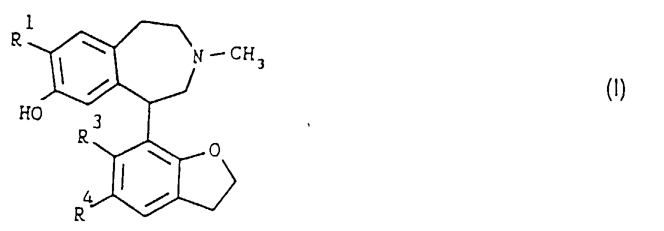 in der R¹ Cl oder Br bedeutet;
R³ und R⁴ unabhängig voneinander Wasserstoff, Halogen, CF₃, CN, NO₂ oder NH₂ bedeuten,
sowie pharmazeutisch verträgliche Säureadditionssalze davon, mit der Maßgabe, daß R³ und R⁴ nicht gleichzeitig Wasserstoff bedeuten können.

2. Verbindungen nach Anspruch 1, worin R¹ die Bedeutung Cl hat.

3. Verbindungen nach Anspruch 1, nämlich
(+)-8-Chlor-5-(5-brom-2,3-dihydrobenzofuran-7-yl)-7-hydroxy-3-methyl-2,3,4,5-tetrahydro-1H-3-benzazepin,
(+)-8-Chlor-5-(2,3-dihydro-5-iodbenzofuran-7-yl)-7-hydroxy-3-methyl-2,3,4,5-tetrahydro-1H-3-benzazepin,
(+)-8-Chlor-5-(5,6-dichlor-2,3-dihydrobenzofuran-7-yl)-7-hydroxy-3-methyl-2,3,4,5-tetrahydro-1H-3-benzazepin,
(+)-8-Chlor-5-(5-chlor-2,3-dihydrobenzofuran-7-yl)-7-hydroxy-3-methyl-2,3,4,5-tetrahydro-1H-3-benzazepin,
8-Chlor-5-(5-nitro-2,3-dihydrobenzofuran-7-yl)-7-hydroxy-3-methyl-2,3,4,5-tetrahydro-1H-3-benzazepin oder
8-Chlor-5-(5-amino-2,3-dihydrobenzofuran-7-yl)-7-hydroxy-3-methyl-2,3,4,5-tetrahydro-1H-3-benzazepin.

4. Pharmazeutische Zusammensetzung, enthaltend eine Verbindung nach den Ansprüchen 1-3 oder ein pharmazeutisch verträgliches Säureadditionssalz davon und einen pharmazeutisch verträglichen Träger oder Verdünnungsmittel.

5. Pharmazeutische Zusammensetzung zur Behandlung einer Erkrankung des Zentralnervensystems, umfassend eine Verbindung nach Anspruch 1-3 zusammen mit einem pharmazeutisch verträglichen Trägerstoff oder Verdünnungsmittel.

6. Pharmazeutische Zusammensetzung nach Anspruch 4 oder 5, die in Form einer oralen Dosierungseinheit mit einem Gehalt an 0,1-100 mg Wirkstoff vorliegt.

7. Verwendung einer Verbindung nach den Ansprüchen 1-3 zur Herstellung einer pharmazeutischen Zusammensetzung zur Behandlung einer Indikation im Zusammenhang mit einer Erkrankung des Zentralnervensystems.

8. Verfahren zur Herstellung einer Verbindung nach Anspruch 1, gekennzeichnet durch
a) die Halogenierung einer Verbindung der Formel II 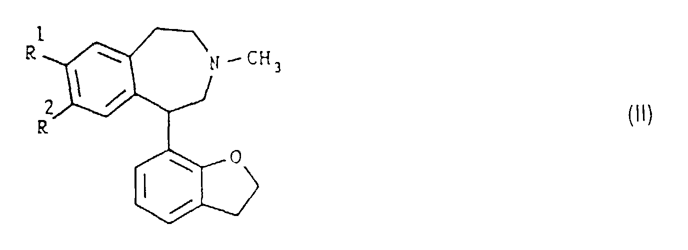 in der R¹ die vorstehend definierte Bedeutung hat und R² die Bedeutung O-C₁₋₄-Alkyl oder O-CO-C₁₋₄-Alkyl hat, unter Bildung einer Verbindung der allgemeinen Formel III 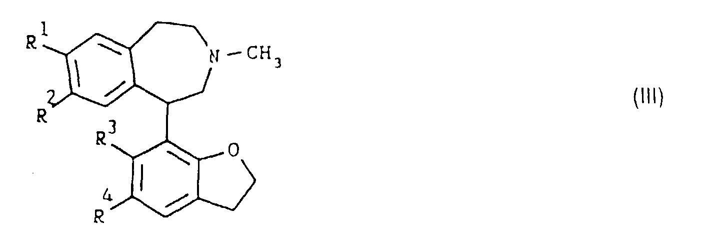 in der R¹ und R² die vorstehend definierten Bedeutungen haben, R³ Halogen oder H bedeutet und R⁴ Halogen bedeutet, und die Desacylierung und Desalkylierung einer Verbindung der Formel III unter Bildung einer Verbindung der Formel I, in der R¹, R³ und R⁴ die vorstehend definierten Bedeutungen haben,
b) die Nitrierung einer Verbindung der Formel II 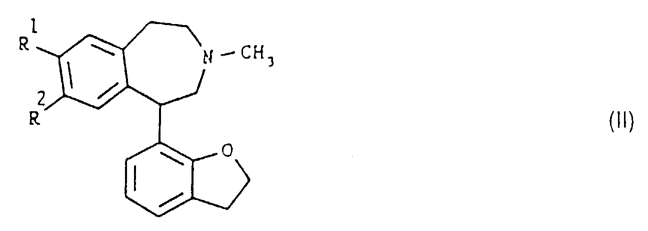 gemäß der Definition in a) unter Bildung einer Verbindung der allgemeinen Formel III 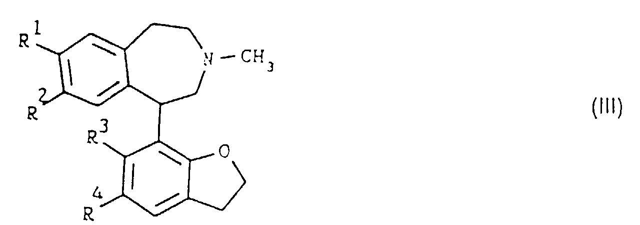 in der R¹ und R² die unter a) definierten Bedeutungen haben, R³ die Bedeutung H hat und R⁴ die Bedeutung -NO₂ hat, und die Desacylierung und Desalkylierung einer Verbindung der Formel III unter Bildung einer Verbindung der Formel I, in der R¹, R³ und R⁴ die vorstehend definierten Bedeutungen haben, oder
c) die Reduktion oder katalytische Hydrierung einer Verbindung der Formel IV 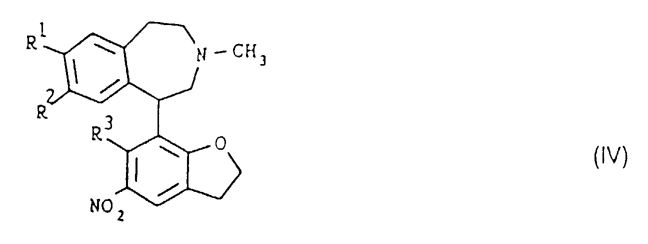 in der R¹ die vorstehend definierte Bedeutung hat, R² die Bedeutung O-C₁₋₄-Alkyl oder O-CO-C₁₋₄-Alkyl hat und R³ die Bedeutung H hat, zu einer Verbindung der Formel III 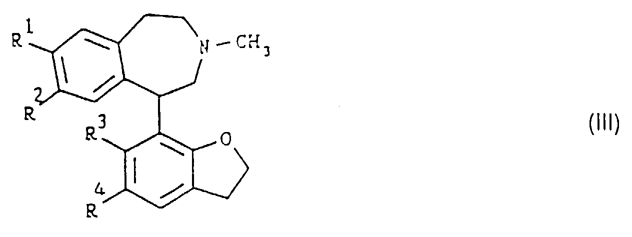 in der R¹, R² und R³ die vorstehend definierten Bedeutungen haben und R⁴ -NH₂ bedeutet und die Desacylierung oder Desalkylierung der Verbindung der Formel III unter Bildung einer Verbindung der Formel I, in der R¹, R³ und R⁴ die vorstehend definierten Bedeutungen haben.

## Revendications

1. 2,3,4,5-tétrahydro-1H-3-benzazépines correspondant à la formule générale I 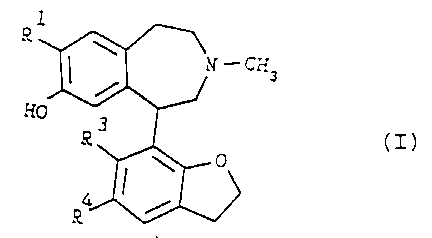 dans laquelle R¹ est Cl ou Br;
R³ et R⁴ sont indépendamment l'hydrogène, un halogène, CF₃, CN, NO₂ ou NH₂ et leurs sels d'addition acide acceptables du point de vue pharmaceutique à condition que R³ et R⁴ ne représentent pas un hydrogène en même temps.

2. Composés selon la revendication 1, dans lesquels R¹ est CI.

3. Un composé selon la revendication 1, qui est la (+)-8-chloro-5-(5-bromo-2,3-dihydrobenzofuran-7-yl)-7-hydroxy-3-méthyl-2,3,4,5-tétrahydro-1H-3-benzazépine, la (+)-8-chloro-5(2,3-dihydro-5-iodo-benzofuran-7-yl)-7-hydroxy-3-méthyl-2,3,4,5-tétrahydro-1H-3-benzazépine, la (+)-8-chloro-5(5,6-dichloro-2,3-dihydrobenzofuran-7-yl)-7-hydroxy-3-méthyl-2,3,4,5-tétrahydro-1H-3-benzazépine, la (+)-8-chloro-5(5-chloro-2,3-dihydrobenzofuran-7-yl)-7-hydroxy-3-méthyl-2,3,4,5-tétrahydro-1H-3-benzazépine, la 8-chloro-5(5-nitro-2,3-dihydrobenzofuran-7-yl)-7-hydroxy-3-méthyl-2,3,4,5-tétrahydro-1H-3-benzazépine ou la 8-chloro-5(5-amino-2,3-dihydrobenzofuran-7-yl)-7-hydroxy-3-méthyl-2,3,4,5-tétrahydro-1H-3-benzazépine.

4. Une composition pharmaceutique comprenant un composé selon les revendications 1 à 3 ou son sel d'addition acide acceptable du point de vue pharmaceutique et un véhicule ou diluant acceptable du point de vue pharmaceutique.

5. Une composition pharmaceutique appropriée pour être utilisée dans le traitement d'une maladie du système nerveux central comprenant un composé selon les revendications 1 à 3 conjointement avec un véhicule ou diluant acceptable du point de vue pharmaceutique.

6. Une composition pharmaceutique selon la revendication 4 ou 5, dans laquelle elle se présente sous la forme d'une unité de dosage oral renfermant de 0,1 à 100 mg de composé actif.

7. Utilisation d'un composé selon les revendications 1 à 3 pour produire une composition pharmaceutique pour le traitement d'une indication relative à une maladie du système nerveux central.

8. Un procédé pour la préparation d'un composé selon la revendication 1, caractérisé par:
a) l'halogénation d'un composé de formule II 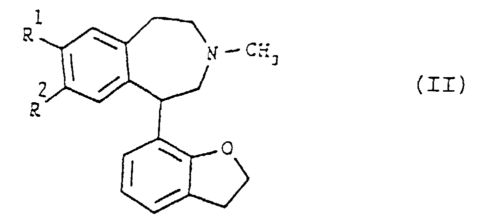 dans laquelle R¹ est défini comme ci-dessus et R² est un O-C₁₄-alkyle ou un O-CO-C₁₄-alkyle pour former un composé de formule générale III 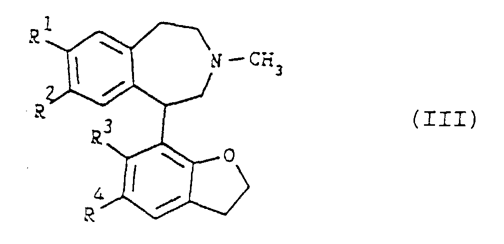 dans laquelle R¹ et R² sont définis comme ci-dessus, R³ est un halogène ou H, R⁴ est un halogène et la désacylation ou la désalkylation d'un composé de formule III pour former un composé de formule I, dans laquelle R¹, R³ et R⁴ sont définis comme ci-dessus,
b) la nitration d'un composé de formule II 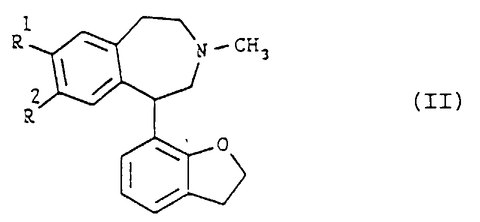 comme défini dans a) pour former un composé de formule générale III 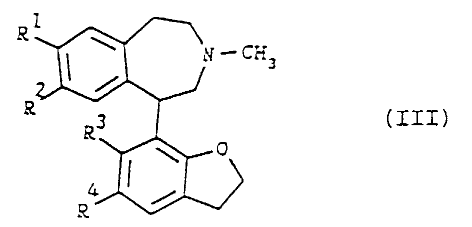 dans laquelle R¹ et R² sont définis comme en a), R³ est H, et R⁴ est -NO₂ et la désacylation ou la désalkylation d'un composé de formule III pour former un composé de formule I, dans laquelle R¹, R³ et R⁴ sont définis comme ci-dessus, ou
c) la réduction ou l'hydrogénation catalytique d'un composé de formule IV, 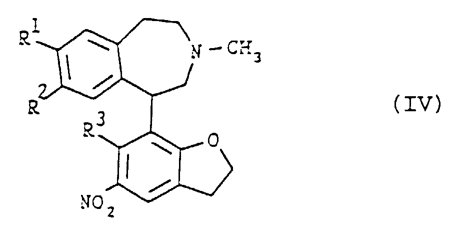 dans laquelle R¹ est défini comme ci-dessus, R² est un O-C₁₄-alkyle ou un O-CO-C₁₄-alkyle et R³ est H, selon un composé de formule III, 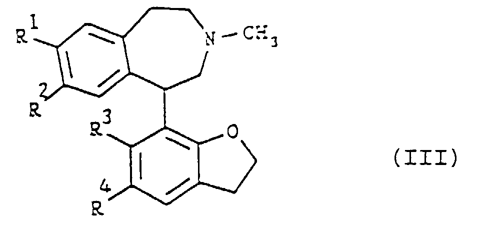 dans laquelle R¹, R² et R³ sont définis comme ci-dessus, et R⁴ est -NH₂, et la désacylation ou la désalkylation du composé de formule III pour former un composé de formule I dans laquelle R¹, R³ et R⁴ sont définis comme ci-dessus.
